(19) **Europäisches Patentamt**
**European Patent Office**
**Office européen des brevets**

(11) **EP 1 564 285 B1**

(12) **EUROPEAN PATENT SPECIFICATION**

(45) Date of publication and mention
of the grant of the patent:
**16.03.2011 Bulletin 2011/11**

(51) Int Cl.:
*C12N 5/00* *(2006.01)*    *C12P 21/08* *(2006.01)*

(21) Application number: **03757654.3**

(22) Date of filing: **22.10.2003**

(86) International application number:
**PCT/CU2003/000012**

(87) International publication number:
**WO 2004/038010 (06.05.2004 Gazette 2004/19)**

(54) **METHOD OF OBTAINING A RECOMBINANT MAMMALIAN MYELOMA CELL LINES ADAPTED TO GROWTH IN SERUM- AND PROTEIN-FREE MEDIA**

VERFAHREN ZUR GEWINNUNG EINER REKOMBINANTEN SÄUGER- MYELOMA ZELLLINIE ADAPTIERT AN WACHSTUM IN SERUM- UND PROTEINFREIEN MEDIEN

METHODE D'OBTENTION DE LIGNEES CELLULAIRES DE MYELOME MAMMIFÈRES RECOMBINANTES ADAPTÉES AU MILIEU EXEMPT DE SERUM ET DE PROTEINES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **23.10.2002  CU 2392002**

(43) Date of publication of application:
**17.08.2005  Bulletin 2005/33**

(73) Proprietor: **Centro de Inmunologia Molecular 12100 Ciudad Habana (CU)**

(72) Inventors:
• **PEREZ RODRIGUEZ, Rolando 10500 Ciudad Habana (CU)**
• **CASTILLO VITLLOCH, Adolfo 13600 Ciudad Habana (CU)**
• **VITORES SARAZOLA, Svieta Santa Fe, Playa, 12200 Ciudad Habana (CU)**

• **BOGGIANO AYO, Tammy o. Flores, Playa, 12100 Ciudad Habana (CU)**
• **ROJAS DEL CALVO, Luis 12100 Ciudad Habana (CU)**

(74) Representative: **Bassil, Nicholas Charles et al Kilburn & Strode LLP 20 Red Lion Street London WC1R 4PJ (GB)**

(56) References cited:
**AU-A- 2 986 484    US-A- 6 100 061**

• **'Hybridoma-SFM: Serum-Free and Protein-Free Media for Hybridoma Culture.' DATA SHEET FROM GIBCO INVITRON CORPORATION, [Online] 08 January 2003, Retrieved from the Internet: <URL:http://invitrogen.com/ contents/sfs/man uals/3913.pdf>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the Invention:**

The present invention relates to biotechnology, specifically to a method of recovering stable cell clones adapted to serum- and protein-free medium by a two-stage adaptation process.

**Background of the invention:**

Since the development of the in vitro cultivation of mammalian cells the demand for large scale production of these cells has increased due to diagnostic and therapeutic potential of many of the products they produce. These useful agents include monoclonal antibodies, human growth hormone, lymphokines, erythropoietin, blood clotting factors and tissue plasminogen activators.

The use of recombinant monoclonal antibodies (rMab) for therapy and *in vivo* diagnosis of different diseases imply in many cases the use of high dose treatments. This fact makes necessary the production of large amount of the rMab of interest with a very high purity.

Several recombinant monoclonal antibodies with potential use in cancer and autoimmune diseases therapy and diagnostic have been expressed in NSO myeloma cells at Center of Molecular Immunology. US 5,891,996, describe the obtainment of the chimeric and humanized antibodies against Epidermal Growth Factor receptor (EGF-R), useful in diagnosis and therapy of tumors expressing said receptor. WO 97/19111 describes anti-CD6 monoclonal antibodies useful in diagnosis and therapy in patients suffering psoriasis. Gavilondo et al. in Hybridoma 9 No.5, 1999 reported an anti-CD3 monoclonal antibody called IOR-T3a

For protein-free culturing conditions, various techniques have been developed. Thus, specifically defined, complete protein-free media have been developed which allow the cell growth under protein-free conditions. WO 97/05240 describes the expression of recombinant proteins under protein-free conditions.

JP 2696001 describes the use of a protein-free medium for the production of factor VIII in CHO cells by adding a non-ionic surface-active agent or cyclodextrin to increase the productivity of the host cells. To increase the effectiveness of these additives, the addition of, e.g., butyrate and lithium is recommended.

WO 96/26266 describes the culturing of cells in a medium which contains a glutamin-containing protein hydrolysate whose content of free amino acids is less than 15% of the total weight of the protein, and whose peptides have a molecular weight of less than 44 kD. As the culturing medium for the cell cultures, a synthetic minimum medium is used as the basic medium to which, inter alia, fetal calf serum, gentamycine and mercapto-ethanol are added in addition to protein hydrolysate. The use of this serum-containing medium for the recombinant production of blood factors has not been mentioned.

U.S. Pat. No. 5,393,668 A describes special synthetic surfaces which allow the growth of adherent cells under protein-free conditions.

To stimulate cell proliferation, CHO cells which over express human insulin have been multiplied on an artificial substrate to which insulin is covalently bound (Ito et al. 1996 PNAS U.S.A. 93:3598-3601).

Reiter et al. (1992. Cytotechnology 9:247-253) describe the immobilisation of r-CHO cells first grown in serum-containing medium at a high density on carriers, and subsequent perfusion of the immobilized cells in protein-free medium during the production phase, wherein a continuous liberation of protein into the cell culture supernatant was found. There, the cells were maintained for less than 10 generations in protein-free medium.

Previous methods for the successful preparation of a large-scale cell culture under protein-free conditions have been described for continuous cell lines; in particular VERO cells (WO 96/15231). There, the cells are grown under serum- and protein-free conditions from the original ampoule up to a large technical scale of 1200 liters.

To adapt cells initially grown under serum-containing conditions to protein free medium is a rather troublesome process which usually takes long time; in addition, it has repeatedly been found that the yield of expressed protein and the productivity of recombinant CHO cells greatly drops after adaptation in protein-free medium as compared to serum-containing conditions (Paterson et al. 1994. Appl. Microbiol. Biotechnol. 40:691-658). This is the consequence of an instability or reduced growth of the recombinant clones due to the changed culturing conditions. Despite the use of a stable original clone, on account of the altered fermentation conditions, repeatedly a large portion of the cells become cells with reduced expression or also non-producers, which overgrow product producers during the production process, whereby the culture of the fermenter finally largely consists of non-producers or of such cells having a low expression.

In the present invention we have established an approach to develop stable cell lines adapted to serum- and protein-free media. Following this approach several clones were isolated in protein-free medium.

**Detailed Description of the Invention:**

*Two stage adaptation of cell lines to protein free medium.*

This procedure comprises recombinant mammalian myeloma cell lines, for which it's not possible to carry out a direct procedure of adaptation from serum-supplemented or serum-free medium to protein-free medium.

The method of the present invention consists of a two stages process during adaptation of cell lines to protein-free medium (PFM).

The first step which is consider as a Non critical stage: the reduction of protein contents occurs without the lost of cell viability and there is not an important decrease of population doubling time in each step of protein concentration. The non-critical stage is observed usually between 5 and 0.5 mg/mL of total protein concentration in the culture medium and the culture shows almost the same growth rate that in the initial culture medium.

This first stage starts with cell line viability between 80 and 100% and cells are grown in culture media with consecutive protein concentration reduction up to a critical protein concentration at which cell viability drop to 0%. This protein concentration is the start point for the next stage.

The second step which is consider as a Critical stage: At this stage it occurs a decrease in cell viability and population-doubling time of the cells and it will take more time to adapt from one step of protein concentration to another. There exist critical protein concentrations in the culture medium, which is not possible to bypass during the adaptation process. These critical protein concentrations are specific for each recombinant cell line, but usually are below 0.6 mg/mL. Once the cells have recovered the initial viability and growth rate at these critical protein concentrations there is possible to subculture to the follow condition with lower protein concentration.

Once the critical protein concentration is fixed, its closest higher protein concentration which support cells growth is consider the pre-critical protein concentration. Starting from the pre-critical protein concentration it is reduced slowly up to cells recover the initial viability and growth rate.

The selected combination of steps to reduce the protein concentration at the critical stage will determine the total adaptation time in this stage and the rate of adaptation ($V_{adapt.}$), calculated as the relationship:

$$V_{adapt.} = \frac{\Delta \text{ Protein concentration}}{\Delta T_{adapt.}}$$

However this step combination will not have influence upon the time needed for adaptation to each protein concentration, including critical concentrations.

In order to determine the end of non-critical stage and the critical protein concentrations it's necessary to carry out an stepwise adaptation, by serial dilutions of the cell culture in the desired protein free medium reducing two fold the protein concentration each time (Table 1). This reduction can be done by the decrease of serum concentration or supplementing the basal medium with different levels of some rich in proteins serum substitute.

*Table 1: Stepwise reduction of protein concentration in order to determine critical concentrations starting from a culture medium supplemented with 5 mg/mL of protein (equivalent to 10 % of fetal bovine serum).*

| Step Number | Total protein concentration mg/mL | Equivalent FBS concentration*, % v/v |
|---|---|---|
| 1 | 5.000 | 10.00 |
| 2 | 2.500 | 5.00 |
| 3 | 1.250 | 2.50 |
| 4 | 0.625 | 1.25 |
| 5 | 0.312 | 0.60 |
| 6 | 0.156 | 0.30 |

(continued)

| Step Number | Total protein concentration mg/mL | Equivalent FBS concentration*, % v/v |
|---|---|---|
| 7 | 0.000 | 0.00 |

| Legend: FBS- Fetal Bovine Serum<br>PFM- Protein Free Medium<br>SCM- Serum Containing Medium<br>* The total protein content of the fetal bovine serum is considered about 50 mg/mL. |

Before starting the adaptation procedure the cells should be maintained with more than 80 % of viability in T-flasks in the standard medium usually employed to culture the cells.

The adaptation process is carried out step by step following the stages described below.

i. Seed 3 wells in the six-well culture plate with recombinant mammalian myeloma cell line using the standard cell culture medium (with the initial protein concentration). The cell density should be in the range of 1 to $5 \times 10^5$ cells/mL. After 48 hours a half of the supernatant is replaced by fresh protein-free medium, thus rendering a final protein concentration which is 50% of the starting condition.

ii. Each 48 hours the supernatant is completely replaced by fresh culture medium with a protein concentration which is 50% of the starting condition.

iii. Cells are grown to confluence in the medium with 50% of the initial protein concentration.

iv. Cells from step iii are seeded in at least 3 wells at a density in the range 1 to $5 \times 10^5$ cells/mL in culture medium with a protein concentration which is 50% of the starting condition. After 48 hours a half of the supernatant is replaced by fresh protein-free medium, rendering a final protein concentration which is 50% of the former condition.

v. Each 48 hours the supernatant is completely replaced by fresh culture medium with a protein concentration which is 50% of the former condition.

vi. Cells are grown to confluence in medium containing 50% of the previous protein concentration.

vii. Steps from (iv) to (vi) are repeated, during each cycle the protein concentration is reduced to 50% of the concentration of the previous cycle. This procedure is repeated up to reach a protein concentration which causes cell death.

viii. Cells are seeded from a cell culture with a viability of 80% or higher growing in the pre-critical protein concentration in at least 3 wells at a density in a range of 2 to $6 \times 10^5$ cells/mL. Cells are grown in the pre-critical protein concentration and after 48 hours, 25% of the supernatant is replaced by fresh protein-free medium, thus rendering a final protein concentration which is the 75% of the pre-critical protein concentration.

ix. Each 48 hours the supernatant is completely replaced by fresh culture medium with a protein concentration which is 75% of the pre-critical protein concentration.

x. Cells are grown to confluence in medium containing 75% of the pre-critical protein concentration.

xi. Cell line derived from the previous step are seeded in at least 3 wells at a density in the range 2 to $6 \times 10^5$ cells/mL in medium containing 75% of the pre-critical protein concentration and after 48 hours, 25% of the culture supernatant is replaced by fresh protein-free medium, thus rendering a final protein concentration which is 75% of the previous concentration.

xii. Each 48 hours the supernatant is completely replaced by fresh culture medium with a protein concentration which is 75% of the previous concentration.

xiii. Cells are grown to confluence in medium containing 75% of the previous protein concentration.

xiv. Steps from (xi) to (xiii) are repeated, reducing the protein concentration each cycle up to 75% of that used in the previous cycle, reaching a protein concentration such that when cells are transferred to a lower concentration they grow with a similar viability and doubling time as the original, so that the subsequent decrease of the protein concentration does not affect the viability or the doubling time.

In the procedure of the present invention the initial culture medium contains a range between 5 to 10% of fetal bovine serum.

The recombinant mammalian cell line adapted to growth in protein-free medium is a myeloma, particularly NSO cells.

The present invention also could be useful when using NSO cell line transfected with a polypeptide or a recombinant protein, particularly when they are transfected with a sequence encoding a recombinant antibody or fragments thereof. The recombinant mammalian myeloma cell lines modified by procedure of the present invention growing in protein-free medium are also disclosed.

Included in this disclosure, but not part of the invention, is any recombinant mammalian myeloma cell line expressing a humanized or chimeric antibody selected from the group consisting of the anti-EGF receptor hR3, anti-CD6 T1hT,anti-CD3 T3Q antibodies or fragments thereof growing in protein-free medium, and thus the antibodies secreted by this cell lines.

The cell lines obtained by the method of the present invention growth in protein-free medium stably for at least 40 generations.

**Examples:**

**Example 1**: Adaptation of recombinant cell line hR3 to protein-free medium.

The recombinant cell line hR3 was obtained by transfection of the myeloma NSO cell line with the vector constructions to express the light and heavy chains of the humanized anti-EGF human receptor hR3 monoclonal antibody.

The adaptation of this cell line to protein-free medium was carried out following the procedure Previously described, by two stage reduction of protein content of the medium.

These cells were cultured in RPMI-1640 medium supplemented with 10 % of FBS. The FBS was replaced by adding the protein reach supplement, Nutridoma NS (Boheringer Manheinn) to RPMI-1640 protein-free medium when the protein concentration was 0.15 mg/mL. The reduction of the protein content in the initial medium was done by successive dilutions with PFHM-II protein-free medium (Gibco).

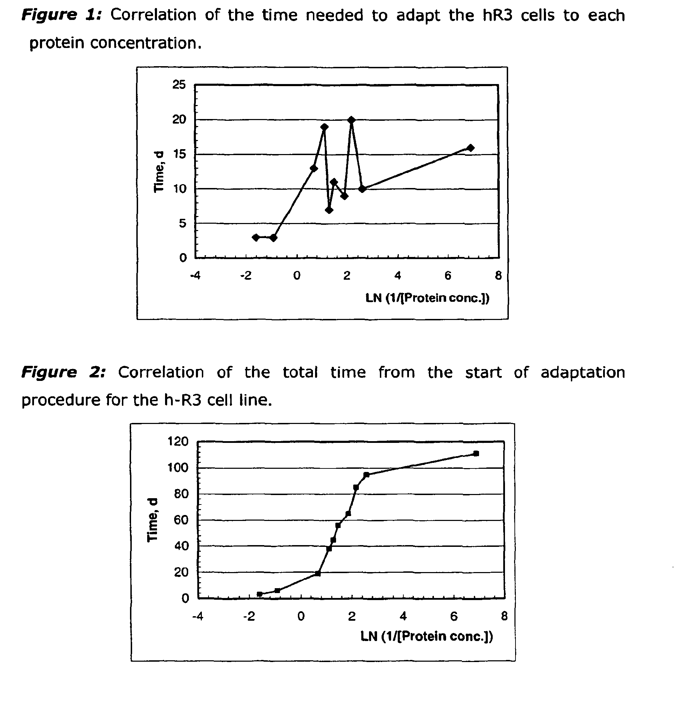

Fig. 1 and 2 respectively show the results of calculating the critical concentrations and adaptation rates $V_{adapt}$.

**Example 2:** Adaptation of recombinant cell line T1hT to protein-free medium.

The recombinant cell line T1hT was obtained by transfection of the myeloma NSO cell line with the vector constructions to express the light and heavy chains of a humanized by epitope T suppression method antihuman CD6 monoclonal antibody.

The adaptation of this cell line to protein/free medium was carried out following the procedure described in the point 2, by two stage reduction of protein content of the medium.

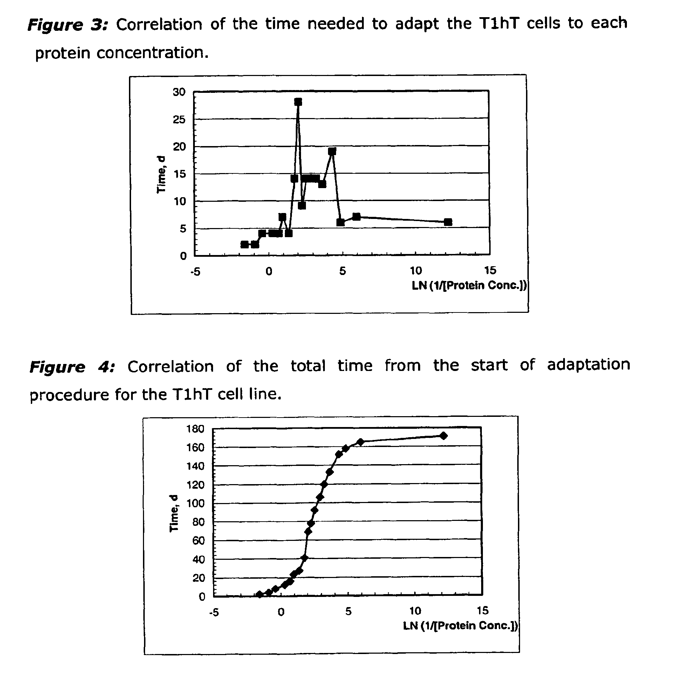

These cells were initially cultured in RPMI-1640 medium supplemented with 10 % of FBS. The reduction in the protein content was done by successive dilution of the initial medium with PFHM-II protein-free medium from Gibco. The results of calculation of the critical concentrations and adaptation rates $V_{adapt}$. are showed in the Fig. 3 and 4 respectively.

**Example 3:** Adaptation of recombinant cell line T3Q to protein-free medium.

The recombinant cell line T3Q was obtained by transfection of the myeloma NSO cell line with the vector constructions to express the light and heavy chains of the humanized monoclonal antibody T3Q which recognize the CD3 receptor on human lymphocytes.

The adaptation of this cell line to protein/free medium was carried out following the procedure described in the point 2, by two stage reduction of protein content of the medium.

These cells were initially cultured in RPMI-1640 medium supplemented with 10 % of FBS. The reduction in the protein content was done by successive dilution in the PFHM-II protein-free medium from Gibco.

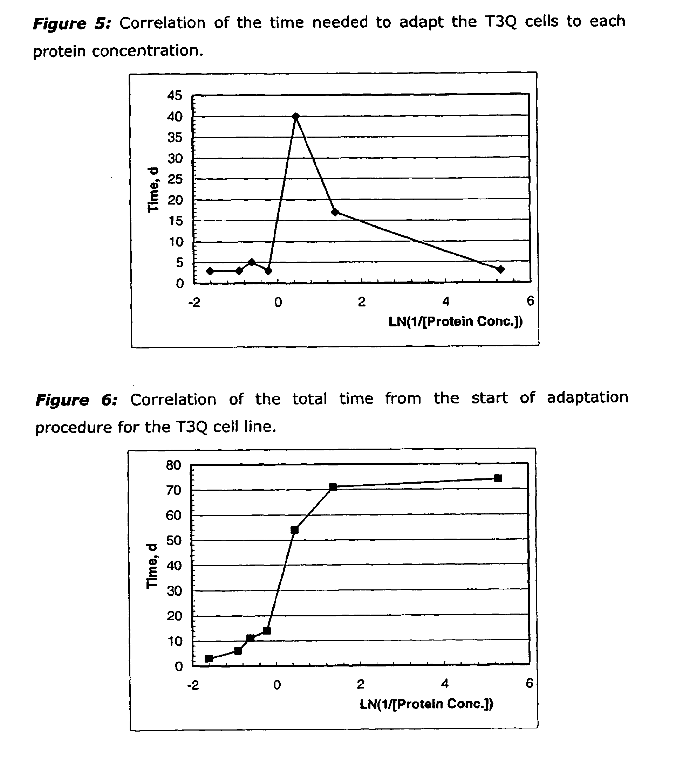

The results of calculation of the critical concentrations and adaptation rates $V_{adapt}$. are showed in the Fig. 5 and 6 respectively.

**Brief Description of the figures:**

*Figure 1*: Correlation of the time needed to adapt the hR3 cells to each protein concentration (up to recover of the viability and doubling time) with the natural logarithm of the inverse of protein concentration. Values of critical concentrations for h-R3 cell line: - 0.32 and 0.11 mg/mL of total protein concentration.

*Figure 2*: Correlation of the total time from the start of adaptation procedure with the natural logarithm of the inverse of protein concentration for the h-R3 cell line. Values of adaptation rates for h-R3 cell line during critical stage - 0.0053 mg/d.

*Figure 3*: Correlation of the time needed to adapt the T1hT cells to each protein concentration (up to recovered he viability and doubling time) with the natural logarithm of the inverse of protein concentration. Values of critical concentrations for T1hT cell line: - 0.12 and 0.01 mg/mL of total protein concentration.

*Figure 4:* Correlation of the total time from the start of adaptation procedure with the natural logarithm of the inverse of protein concentration for the T1hT cell line. Values of adaptation rates for T1hT cell line - 0.0014 mg/d.

*Figure 5*: Correlation of the time needed to adapt the T3Q cells to each protein concentration (after recovering of the viability and doubling time) with the natural logarithm of the inverse of protein concentration. Values of critical concentrations for T3Q cell line: - 0.63 mg/mL of total protein concentration.

*Figure 6:* Correlation of the total time from the start of adaptation procedure with the natural logarithm of the inverse of protein concentration for the T3Q cell line. Values of adaptation rates for T3Q cell line - 0.0172 mg/d.

**Claims**

1. A method for obtaining a recombinant mammalian myeloma cell line adapted to growth in serum- and protein-free media, which comprises two stages:

   I. A first stage wherein the cell line viability is between 80 and 100% and cells are grown in culture media with consecutive protein concentration reduction up to a critical protein concentration at which cell viability drops to 0%; and

   II. A second stage wherein once the critical concentration has been predetermined, then the pre-critical concentration is fixed as such a protein concentration in which cellular growth is possible and is the start point to slowly reduce the protein concentration until the cell culture reaches the initial cellular viability and population doubling time

2. The method according to claim 1 wherein the first stage consists of the following steps:

   i. Seeding 3 wells in the six-well culture plate with recombinant mammalian myeloma cell line using the standard cell culture medium with the initial protein concentration, wherein the cell density is in the range of 1 to 5 x $10^5$ cells/mL and after 48 hours replacing half of the supernatant by fresh protein-free medium, thus rendering a final protein concentration which is 50% of the starting condition;

   ii. Completely replacing every 48 hours the supernatant by fresh culture medium with a protein concentration which is 50% of the starting condition;

   iii. Growing the cells to confluence in the medium with 50% of the initial protein concentration;

   iv. Seeding cells from step iii in at least 3 wells at a density in the range 1 to 5 $\times$ $10^5$ cells/mL in culture medium with a protein concentration which is 50% of the starting condition, replacing half of the supernatant after 48 hours by fresh protein-free medium, rendering a final protein concentration which is 50% of the former condition;

   v. Completely replacing the supernatant every 48 hours by fresh culture medium with a protein concentration which is 50% of the former condition

   vi. Growing the cells to confluence in medium containing 50% of the previous protein concentration; and

   vii. Repeating steps (iv) to (vi), wherein during each cycle the protein concentration is reduced to 50% of the concentration of the previous cycle, until a protein concentration is reached which causes cell death.

3. The method according to claim 1 wherein the second stage consists of the following steps:

   viii. Seeding the mammalian myeloma cells from a cell culture with a viability of 80% or higher growing in the pre-critical protein concentration in at least 3 wells at a density in a range of 2 to 6 x $10^5$ cells/mL, growing cells in the pre-critical protein concentration and after 48 hours replacing 25% of the supernatant by fresh protein-free medium, thus rendering it a final protein concentration which is 75% of the pre-critical protein concentration;

   ix. Completely replacing the supernatant every 48 hours by fresh culture medium with a protein concentration which is 75% of the pre-critical protein concentration;

   x. Growing the cells to confluence in medium containing 75% of the pre-critical protein concentration;

   xi. Seeding cell line derived from the previous step in at least 3 wells at a density in the range 2 to 6 x $10^5$ cells/mL in medium containing 75% of the pre-critical protein concentration and after 48 hours, replacing 25% of the supernatant by fresh protein-free medium, thus rendering it a final protein concentration which is 75% of the previous concentration;

   xii. Completely replacing the supernatant every 48 hours by fresh culture medium with a protein concentration which is 75% of the previous concentration;

   xiii. Growing the cells to confluence in medium containing 75% of the previous protein concentration; and

   xiv. Repeating steps from (xi) to (xiii), reducing the protein concentration each cycle up to 75% of that used in the previous cycle, reaching a protein concentration such that when cells are transferred to a lower concentration they grow with a similar viability and doubling time as the original, so that the subsequent decrease of the protein concentration does not affect the viability or the doubling time.

4. The method according to claims 1 to 3 wherein the serum and protein-containing medium in which the cells are initially seeded comprises between 5% and 10% of fetal bovine serum.

5. The method according to claim 4, wherein the recombinant mammalian myeloma cell line is the NSO cell line.

6. The method according to claim 5 wherein said NSO cell line contains a sequence encoding a recombinant polypeptide or a recombinant protein that codifies for a recombinant antibody or a fragment thereof.

7. The method according to claim 6, wherein said NSO cell line contains a sequence encoding the anti-EGF receptor antibody hR3.

8. Use of the method according to claims 1 to 6 for obtaining a mammalian myeloma cell line adapted to growth in serum and protein-free media.


**Patentansprüche**

1. Verfahren zur Gewinnung einer rekombinanten Säugetier-Myelomzelllinie, die für das Wachstum in Serum- und Protein-freien Medien adaptiert ist, welches zwei Phasen umfasst:

   I. Eine erste Phase bei der die Lebensfähigkeit der Zelllinie zwischen 80 und 100 % liegt und die Zellen in Kulturmedien kultiviert werden und zwar bei fortlaufender Verringerung der Proteinkonzentration bis hin zu einer kritischen Proteinkonzentration bei der die Lebensfähigkeit der Zellen auf 0 % fällt; und
   II. Eine zweite Phase bei der, wenn einmal die kritische Konzentration vorbestimmt worden ist, dann die vorkritische Konzentration als eine solche Proteinkonzentration fixiert wird bei der das zelluläre Wachstum möglich ist und die der Ausgangspunkt für langsames Verringern der Proteinkonzentration ist bis die Zellkultur die anfängliche zelluläre Lebensfähigkeit und Populationsverdopplungszeit erreicht hat.

2. Verfahren gemäß Anspruch 1, wobei die erste Phase aus den folgenden Schritten besteht:

   i. Aussäen von 3 Vertiefungen in der sechs-Well-Kulturplatte mit rekombinanter Säugetier-Myelomzelllinie unter Verwendung des Standard-Zellkulturmediums mit der anfänglichen Proteinkonzentrat, wobei die Zelldichte sich im Bereich von 1 bis 5 x $10^5$ Zellen/mL befindet, und nach 48 Stunden Ersetzen der Hälfte des Überstands durch frisches Protein-freies Medium, wodurch sich eine endgültige Proteinkonzentration ergibt, die 50 % der Ausgangsbeschaffenheit beträgt.
   ii. Vollständiges Ersetzen des Überstands alle 48 Stunden durch frisches Kulturmedium mit einer Proteinkonzentration, die 50 % der Ausgangsbeschaffenheit beträgt.
   iii. Kultivieren der Zellen bis zur Konfluenz in dem Medium mit 50 % der anfänglichen Proteinkonzentration.
   iv. Aussäen von Zellen von Schritt iii in mindestens 3 Vertiefungen bei einer Dichte im Bereich von 1 bis 5 x $10^5$ Zellen/mL in Kulturmedium mit einer Proteinkonzentration, die 50 % von der Ausgangsbeschaffenheit beträgt, Ersetzen der Hälfte des Überstands nach 48 Stunden durch frisches Protein-freies Medium, wodurch sich eine endgültige Proteinkonzentration ergibt, die 50 % der vorherigen Beschaffenheit beträgt;
   v. Vollständiges Ersetzen des Überstands alle 48 Stunden durch frisches Kulturmedium mit einer Proteinkonzentration, die 50 % der vorherigen Beschaffenheit beträgt;
   vi. Kultivieren der Zellen zur Konfluenz in dem Medium, das 50 % der vorherigen Proteinkonzentration enthält; und
   vii. Wiederholen der Schritte (iv) bis (vi), wobei während jedem Durchgang die Proteinkonzentration auf 50 % der Konzentration des vorherigen Durchgangs verringert wird bis eine Proteinkonzentration erreicht wird, die Zelltod verursacht.

3. Verfahren gemäß Anspruch 1, wobei die zweite Phase aus den folgenden Schritten besteht:

   viii. Aussäen der Säugetier-Myelomzellen aus einer Zellkultur mit einer Lebensfähigkeit von 80 % oder höher, die in der vorkritischen Proteinkonzentration in mindestens 3 Vertiefungen bei einer Dichte im Bereich von 2 bis 6 x $10^5$ Zellen/ML wachsen, Kultivieren der Zellen in der vorkritischen Proteinkonzentration und nach 48 Stunden Ersetzen von 25 % des Überstands durch frisches Protein-freies Medium, wodurch sich eine endgültige Proteinkonzentration ergibt, die 75 % der vorkritischen Proteinkonzentration beträgt;

ix. Vollständiges Ersetzen des Überstands alle 48 Stunden durch frisches Kulturmedium mit einer Proteinkonzentration, die 75 % der vorkritischen Proteinkonzentration beträgt;

x. Kultivieren der Zellen zur Konfluenz in dem Medium, das 75 % der vorkritischen Proteinkonzentration enthält;

xi. Aussäen der Zelllinie, die aus dem vorherigen Schritt stammt, in zumindest 3 Vertiefungen bei einer Dichte im Bereich von 2 bis 6 x $10^5$ Zellen/mL in Medium, das 75 % der vorkritischen Proteinkonzentration enthält, und nach 48 Stunden Ersetzen von 25 % des Überstands durch frisches Protein-freies Medium, wodurch sich eine endgültige Proteinkonzentration ergibt, die 75 % der vorherigen Konzentration beträgt;

xii. Vollständiges Ersetzen des Überstands alle 48 Stunden durch frisches Kulturmedium mit einer Proteinkonzentration, die 75 % der vorherigen Konzentration beträgt;

xiii. Kultivieren der Zellen zur Konfluenz in dem Medium, das 75 % der vorherigen Proteinkonzentration enthält; und

xiv. Wiederholen der Schritte von (xi) bis (xiii), Verringern der Proteinkonzentration bei jedem Durchlauf bis zu 75 % von jener im vorherigen Durchlauf verwendeten, Erreichen einer solchen Proteinkonzentration, dass, wenn Zellen auf eine niedrigere Konzentration transferiert werden, sie mit einer ähnlichen Lebensfähigkeit und Verdopplungszeit wachsen wie das Original, sodass die folgende Verringerung der Proteinkonzentration keine Auswirkung auf die Lebensfähigkeit oder die Verdopplungszeit hat.

4. Verfahren gemäß der Ansprüche 1 bis 3, wobei das Serum und das Protein-enthaltende Medium, in das die Zellen zu Anfang gesät werden, zwischen 5 % und 10 % von fötalem Rinderserum enthält.

5. Verfahren gemäß Anspruch 4, wobei es sich bei der rekombinanten Säugetier-Myelomzelllinie um die NSO-Zelllinie handelt.

6. Verfahren gemäß Anspruch 5, wobei die NSO-Zelllinie eine Sequenz enthält, die ein rekombinantes Polypeptid oder ein rekombinantes Protein kodiert, das für einen rekombinanten Antikörper oder ein Fragment davon kodifiziert.

7. Verfahren gemäß Anspruch 6, wobei die NSO-Zelllinie eine Sequenz enthält, die den anti-EGF-Rezeptor-Antikörper hR3 kodiert.

8. Verwendung des Verfahrens gemäß der Ansprüche 1 bis 6 zur Gewinnung einer Säugetier-Myelomzelllinie, die für das Wachstum in Serum- und Protein-freien Medien adaptiert ist.


**Revendications**

1. Méthode d'obtention d'une lignée de cellules recombinantes de myélome de mammifère qui est capable de se développer dans des milieux exempts de sérum et de protéines, comprenant deux stade :

    I. un premier stade dans lequel la viabilité de la lignée de cellules est située entre 80 % et 100 % et les cellules sont cultivées dans des milieux de culture en réduisant successivement la concentration en protéines jusqu'à une concentration critique en protéines à laquelle la viabilité cellulaire chute à 0 % ; et

    II. un second stade dans lequel, après la détermination de la concentration critique, la concentration précritique est alors fixée à une concentration en protéines dans laquelle une croissance cellulaire est possible et est le point de départ pour réduire lentement la concentration en protéines jusqu'à ce que la culture de cellules atteigne la viabilité cellulaire initiale et la durée initiale de doublement de la population.

2. Méthode selon la revendication 1, dans laquelle le premier stade est constitué des étapes suivantes :

    i. l'ensemencement de 3 puits d'une plaque de culture à six puits avec une lignée de cellules recombinantes de myélome de mammifère en utilisant le milieu de culture cellulaire standard avec la concentration initiale en protéines, la densité cellulaire étant située dans la plage allant de 1 à 5 x $10^5$ cellules/ml, et après 48 heures, le remplacement de la moitié du surnageant par un milieu frais exempt de protéines, pour ainsi obtenir une concentration finale en protéines de 50 % par rapport aux conditions de départ ;

    ii. le remplacement total toutes les 48 heures du surnageant par un milieu de culture frais ayant une concentration en protéines de 50 % par rapport aux conditions de départ ;

    iii. la culture des cellules jusqu'à confluence dans le milieu ayant une concentration en protéines de 50 % par rapport aux conditions de départ ;

iv. l'ensemencement des cellules de l'étape iii dans au moins 3 puits à une densité située dans la plage allant de 1 à 5 x $10^5$ cellules/ml dans un milieu de culture ayant une concentration en protéines de 50 % par rapport aux conditions de départ, le remplacement de la moitié du surnageant après 48 heures par un milieu frais exempt de protéines, pour ainsi obtenir une concentration finale en protéines de 50 % par rapport aux conditions précédentes ;

v. le remplacement total du surnageant toutes les 48 heures par un milieu de culture frais ayant une concentration en protéines de 50 % par rapport aux conditions précédentes ;

vi. la culture des cellules jusqu'à confluence dans un milieu contenant 50 % de la concentration précédente en protéines ; et

vii. la répétition des étapes (iv) à (vi), la concentration en protéines étant réduite à 50 % de la concentration du cycle précédent durant chaque cycle, jusqu'à ce que soit atteinte une concentration en protéines provoquant une mort cellulaire.

3. Méthode selon la revendication 1, dans laquelle le second stade est constitué des étapes suivante :

viii. l'ensemencement des cellules de myélome de mammifère provenant d'une culture de cellules présentant une viabilité supérieure ou égale à 80 % effectuée à la concentration précritique en protéines dans au moins 3 puits à une densité allant de 2 à 6 x $10^5$ cellules/ml, la culture des cellules dans la concentration précritique en protéines et, après 48 heures, le remplacement de 25 % du surnageant par un milieu frais exempt de protéines, pour ainsi obtenir une concentration finale en protéines égale à 75 % de la concentration précritique en protéines ;

ix. le remplacement total du surnageant toutes les 48 heures par un milieu de culture frais ayant une concentration en protéines égale à 75 % de la concentration précritique en protéines ;

x. la culture des cellules jusqu'à confluence dans un milieu contenant 75 % de la concentration précritique en protéines ;

xi. l'ensemencement de la lignée de cellules dérivant de l'étape précédente dans au moins 3 puits à une densité située dans la plage allant de 2 à 6 x $10^5$ cellules/ml dans un milieu contenant 75 % de la concentration précritique en protéines et, après 48 heures, le remplacement de 25 % du surnageant par un milieu frais exempt de protéines, pour ainsi obtenir une concentration finale en protéines égale à 75 % de la concentration précédente ;

xii. le remplacement total du surnageant toutes les 48 heures par un milieu de culture frais ayant une concentration en protéines égale à 75 % de la concentration précédente ;

xiii. la culture des cellules jusqu'à confluence dans un milieu contenant 75 % de la concentration précédente en protéines ; et

xiv. la répétition des étapes (xi) à (xiii), en réduisant la concentration en protéines à chaque cycle à 75 % de celle utilisée dans le cycle précédent, pour atteindre une concentration en protéines telle que quand les cellules sont transférées dans une concentration plus basse, leur développement s'effectue avec une viabilité et une durée de doublement similaires à l'original, de sorte que la diminution suivante de la concentration en protéines n'affecte pas la viabilité ou la durée de doublement.

4. Méthode selon les revendications 1 à 3, dans laquelle le milieu contenant du sérum et des protéines dans lequel les cellules sont initialement ensemencées comprend entre 5 % et 10 % de sérum bovin foetal.

5. Méthode selon la revendication 4, dans lequel la lignée de cellules recombinantes de myélome de mammifère est la lignée des cellules NSO.

6. Méthode selon la revendication 5, dans laquelle ladite lignée des cellules NSO contient une séquence codant un polypeptide recombinant ou une protéine recombinant qui codifie un anticorps recombinant ou l'un de ces fragments.

7. Méthode selon la revendication 6, dans laquelle ladite lignée des cellules NSO contient une séquence codant l'anticorps hR3 anti-récepteur EGF.

8. Utilisation de la méthode selon les revendications 1 à 6, pour obtenir une lignée de cellules de myélome de mammifère capable de se développer dans des milieux exempts de sérum et de protéines.

**Figure 1:** Correlation of the time needed to adapt the hR3 cells to each protein concentration.

**Figure 2:** Correlation of the total time from the start of adaptation procedure for the h-R3 cell line.

**Figure 3:** Correlation of the time needed to adapt the T1hT cells to each protein concentration.

**Figure 4:** Correlation of the total time from the start of adaptation procedure for the T1hT cell line.

**Figure 5:** Correlation of the time needed to adapt the T3Q cells to each protein concentration.

**Figure 6:** Correlation of the total time from the start of adaptation procedure for the T3Q cell line.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5891996 A
- WO 9719111 A
- WO 9705240 A
- JP 2696001 B
- WO 9626266 A
- US 5393668 A
- WO 9615231 A

**Non-patent literature cited in the description**

- **Gavilondo et al.** *Hybridoma,* 1999, vol. 9 (5
- **Ito et al.** *PNAS U.S.A.,* 1996, vol. 93, 3598-3601
- **Reiter et al.** *Cytotechnology,* 1992, vol. 9, 247-253
- **Paterson et al.** *Appl. Microbiol. Biotechnol.,* 1994, vol. 40, 691-658